**Europäisches Patentamt**

**European Patent Office**

**Office européen des brevets**

(19)

(11) **EP 1 559 425 A1**

(12) **EUROPEAN PATENT APPLICATION**
published in accordance with Art. 158(3) EPC

(43) Date of publication:
**03.08.2005 Bulletin 2005/31**

(51) Int Cl.⁷: **A61K 31/401**, A61P 17/00, A61P 37/08

(21) Application number: **03809872.9**

(22) Date of filing: **31.10.2003**

(86) International application number:
**PCT/JP2003/014022**

(87) International publication number:
**WO 2004/039368 (13.05.2004 Gazette 2004/20)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **01.11.2002 JP 2002319973**

(71) Applicant: **KYOWA HAKKO KOGYO CO., LTD.**
**Chiyoda-ku, Tokyo 100-8185 (JP)**

(72) Inventors:
• **TAKAHASHI, Tomoya,**
c/o Kyowa Engineering Co., Ltd.
**Chiyoda-ku, Tokyo 102-0074 (JP)**
• **KOBAYASHI, Asako**
**Niigata-shi, Niigata 950-0916 (JP)**

(74) Representative: **VOSSIUS & PARTNER**
**Siebertstrasse 4**
**81675 München (DE)**

(54) **PERORAL PREPARATION FOR PREVENTION OF OR TREATMENT FOR ATOPIC DERMATITIS**

(57)    In accordance with the present invention, a peroral preparation, food and drink, feed, food additive or feed additive for prevention or treatment of atopic dermatitis comprising hydroxyproline or N-acylated derivative of hydroxyproline or a salt thereof is able to be provided and, particularly, said peroral preparation, food and drink, feed, food additive or feed additive comprising 0.1 to 90% by weight of hydroxyproline or N-acylated derivative of hydroxyproline or a salt thereof is able to be provided.

**EP 1 559 425 A1**

**Description**

TECHNICAL FIELD

The present invention relates to peroral preparation, food and drink, feed, food additive and feed additive for prevention or treatment of atopic dermatitis comprising hydroxyproline or N-acylated derivative of hydroxyproline or a salt thereof.

BACKGROUND ART

According to epidemiological survey of the Ministry of Health, Labor and Welfare of Japan, about 30% of the total population is suffering from allergic diseases. Particularly with regard to atopic dermatitis, an increase in prevalence rate and also an increase in intractability have been reported not only in small children but also in adults.

With regard to a therapeutic agent for allergic diseases, antihistaminic agents, steroidal preparations and antiallergic agents are being used at present (refer to *Igaku* no Ayumi, volume 180, number 1, page 70, 1997).

Although antihistaminic agents and antiallergic agents have a quick action with regard to an effect for reduction of itching feel but that is a mere allopathic treatment.

When steroidal agents are used in large quantities or for a long period, there are adverse actions such as induction of infectious diseases, reduction in adrenocortical function, dilation of capillary vessels and skin atrophy and, therefore, they are not safe therapeutic agents.

With regard to drugs or food and drink having preventive or therapeutic effect for atopic dermatitis, oolong tea extract (refer to Japanese Published Unexamined Patent Application no. 77231/1998), raffinose (refer to Food *Style 21,* volume 2, number 4, pages 262-265, 1998), propolis (refer to Food *Style 21*, volume 2, number 4, pages 55-56, 1998), etc. have been reported.

It has been known that the external application of hydroxyproline or N-acylated derivative of hydroxyproline or a salt thereof achieves a preventive or therapeutic effect for atopic dermatitis (refer to WO 02/06225 pamphlet). Antirheumatic agent and wound therapeutic agent compounded with hydroxyproline or N-acylated derivative of hydroxyproline or a salt thereof have been known as well (refer to Japanese Published Unexamined Patent Application no.337526/1996). However, it has not been known yet that hydroxyproline or N-acylated derivative of hydroxyproline or a salt thereof to be taken by mouth achieves a preventive or therapeutic effect for atopic dermatitis.

DISCLOSURE OF THE INVENTION

An object of the present invention is to provide a safe peroral preparation, food and drink, feed, food additive and feed additive for prevention or treatment of atopic dermatitis.

The present invention relates to the following (1) to (14).

(1) A peroral preparation for prevention or treatment of atopic dermatitis comprising hydroxyproline or N-acylated derivative of hydroxyproline or a salt thereof.

(2) The peroral preparation according to the above (1), wherein it comprises 0.1 to 90% by weight of hydroxyproline or N-acylated derivative of hydroxyproline or a salt thereof.

(3) The peroral preparation according to the above (1) or (2), wherein the acyl group of N-acylated derivative of hydroxyproline is an acyl group having 1 to 24 carbon atoms.

(4) The peroral preparation according to any one of the above (1) to (3), wherein the N-acylated derivative of hydroxyproline is N-acetylated derivative, N-propionylated derivative, N-butyrylated derivative or isobutyrylated derivative.

(5) Food and drink or feed for prevention or treatment of atopic dermatitis comprising hydroxyproline or N-acylated derivative of hydroxyproline or a salt thereof.

(6) The food and drink or feed according to the above (5), wherein it comprises 0.1 to 90% by weight of hydroxyproline or N-acylated derivative of hydroxyproline or a salt thereof.

(7) The food and drink or feed according to the above (5) or (6), wherein the acyl group of N-acylated derivative of hydroxyproline is an acyl group having 1 to 24 carbon atoms.

(8) The food and drink or feed according to any one of the above (5) to (7), wherein the N-acylated derivative of hydroxyproline is N-acetylated derivative, N-propionylated derivative, N-butyrylated derivative or isobutyrylated derivative.

(9) Food additive or feed additive for prevention or treatment of atopic dermatitis comprising hydroxyproline or N-acylated derivative of hydroxyproline or a salt thereof.

(10) The food additive or feed additive according to the above (9), wherein it comprises 0.1 to 90% by weight of hydroxyproline or N-acylated derivative of hydroxyproline or a salt thereof.

(11) The food additive or feed additive according to the above (9) or (10), wherein the acyl group of N-acylated derivative of hydroxyproline is an acyl group having 1 to 24 carbon atoms.

(12) The food additive or feed additive according to any one of the above (9) to (11), wherein the N-acylated derivative of hydroxyproline is N-acetylated derivative, N-propionylated derivative, N-butyrylated derivative or isobutyrylated derivative.

(13) A method of preventing or treating atopic dermatitis, which comprises orally administering or ingesting hydroxyproline or N-acylated derivative of hydroxyproline or a salt thereof in an effective amount.

(14) Use of hydroxyproline or N-acylated derivative of hydroxyproline or a salt thereof for the manufacture of peroral preparation, food, feed, food additive or feed additive for prevention or treatment of atopic dermatitis.

The hydroxyproline used in the present invention may be any stereoisomer of hydroxyproline. Thus, there are eight kinds of stereoisomers depending upon the fact whether proline in hydroxyproline is D- or L-isomer, whether position of hydroxyl group is at 3- or 4-position and whether the stereoisomer is cis or trans and any of such compounds may be used in the present invention.

With regard to specific hydroxyproline, mention may be made of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

Hydroxyproline is a kind of amino acid which is widely present in nature as a main constituting amino acid component of collagen and also as a constituting amino acid for elastin and is able to be produced, for example, by hydrolysis of collagen derived from animals such as swine, cattle, etc. with an acid followed by purification according to a conventional method.

trans-4-Hydroxy-L-proline is able to be produced using an enzyme which hydroxylates a 4-position of proline (Japanese Published Unexamined Patent Application no.313179/1995) isolated from the genus *Amycolatopsis* or the genus *Dactylosporangium*. cis-3-Hydroxy-L-proline is able to be produced using an enzyme which hydroxylates a 3-position of proline (Japanese Published Unexamined Patent Application no.322885/1995) isolated from the genus *Streptomyces* (*Bioindustry,* volume 14, number 31, 1997).

The above-mentioned hydroxyproline produced using an enzyme derived from microorganisms has an excellent quality and is more preferred as hydroxyproline used in the present invention.

With regard to the N-acylated derivative of hydroxyproline used in the present invention, N-acylated derivatives of the above-mentioned various stereoisomers of hydroxyproline may be exemplified. Examples of the acyl group of the N-acylated derivatives include acyl group having 1 to 24 carbon atoms, more preferably 1 to 12 carbon(s) and, particularly preferably, 1 to 6 carbon(s). Specific examples include formyl, acetyl, propionyl, butyryl, isobutyryl, valeryl, pivaloyl, hexanoyl, heptanoyl, octanoyl, nonanoyl, decanoyl, undecanoyl, dodecanoyl and the like and particularly preferred ones are acetyl, propionyl, butyryl and isobutyryl.

Examples of salt of hydroxyproline or of the N-acylated derivative of hydroxyproline include salt with alkaline metal such as sodium, potassium, lithium, etc., salt with alkaline earth metal such as calcium, magnesium, etc., ammonium salt, addition salt with amine such as monoethanolamine, diethanolamine, triethanolamine, triisopropanolamine, etc., and addition salt with basic amino acid such as arginine, lysine, etc. or the like.

The N-acylated derivative of hydroxyproline is able to be produced by a known method. For example, the N-acylated derivative of hydroxyproline is able to be produced in such a manner that a linear or branched and saturated or unsaturated fatty acid having 1 to 24 carbon atoms is converted to a halide such as chloride, bromide, etc. using a halogenating agent such as thionyl chloride, phosgene, etc. and then condensed with hydroxyproline or that fatty acid is converted to acid anhydride and then made to react with hydroxyproline.

With regard to the fatty acid, a fatty acid such as formic acid, acetic acid, propionic acid, butyric acid, isobutyric acid, valeric acid, isovaleric acid, pivalic acid, hexanoic acid, heptanoic acid, octanoic acid, nonanoic acid, decanoic acid, undecanoic acid, dodecanoic acid, etc. is used either solely or as a mixture.

A method for the production of N-acylated derivative of hydroxyproline via an acid halide is exemplified as follows.

A fatty acid is dispersed in a solvent such as methylene chloride, chloroform, carbon tetrachloride, benzene, toluene, xylene, n-hexane, etc. and 1 to 5 equivalent(s) of a halogenating agent is added thereto followed by being made to react to give a fatty acid halide. Thereafter, hydroxyproline is dissolved or dispersed in a solvent and, while the resulting solution is kept at 5 to 70°C, the above fatty acid halide is added in an amount of 0.3 to 3.0 equivalent (s) to hydroxyproline to conduct an acylation reaction whereupon an N-acylated derivative of hydroxyproline is produced.

Examples of the solvent used for the acylation reaction include water, methanol, ethanol, isopropanol, isobutanol, acetone, toluene, tetrahydrofuran, ethyl acetate, N,N-dimethylformamide, dimethyl sulfoxide, etc. and each of them may be used solely or as a mixture. In dissolving or dispersing hydroxyproline in a solvent, 0.8 to 2.0 equivalent (s) of alkaline substance such as sodium hydroxide, potassium hydroxide, etc. may be dissolved or dispersed in a solvent if necessary.

In case of obtaining a salt of the N-acyl derivative of hydroxyproline, the product itself may be purified when an N-acylated derivative of hydroxyproline is prepared in a form of salt. When it is prepared in a free form, it may be dissolved or suspended in an appropriate solvent and a base is added thereto to form a salt.

With regard to purification, a conventional method such as crystallization, chromatography, etc. may be used.

Specific examples of the N-acylated derivative of hydroxyproline include N-acetyl-cis-4-hydroxy-L-proline,
N-acetyl-cis-4-hydroxy-D-proline,
N-acetyl-cis-3-hydroxy-L-proline,
N-acetyl-cis-3-hydroxy-D-proline,
N-acetyl-trans-4-hydroxy-L-proline,
N-acetyl-trans-4-hydroxy-D-proline,
N-acetyl-trans-3-hydroxy-L-proline,
N-acetyl-trans-3-hydroxy-D-proline,
N-propionyl-cis-4-hydroxy-L-proline,
N-propionyl-cis-4-hydroxy-D-proline,
N-propionyl-cis-3-hydroxy-L-proline,
N-propionyl-cis-3-hydroxy-D-proline,
N-propionyl-trans-4-hydroxy-L-proline,
N-propionyl-trans-4-hydroxy-D-proline,
N-propionyl-trans-3-hydroxy-L-proline,
N-propionyl-trans-3-hydroxy-D-proline,
N-butyryl-cis-4-hydroxy-L-proline,
N-butyryl-cis-4-hydroxy-D-proline,
N-butyryl-cis-3-hydroxy-L-proline,
N-butyryl-cis-3-hydroxy-D-proline,
N-butyryl-trans-4-hydroxy-L-proline,
N-butyryl-trans-4-hydroxy-D-proline,
N-butyryl-trans-3-hydroxy-L-proline,
N-butyryl-trans-3-hydroxy-D-proline,
N-isobutyryl-cis-4-hydroxy-L-proline,
N-isobut-yryl-cis-4-hydroxy-D-proline,
N-isobutyryl-cis-3-hydroxy-L-proline,
N-isobutyryl-cis-3-hydroxy-D-proline,
N-isobutyryl-trans-4-hydroxy-L-proline,
N-isobutyryl-trans-4-hydroxy-D-proline,
N-isobutyryl-trans-3-hydroxy-L-proline,
N-isobutyryl-trans-3-hydroxy-D-proline and the like.

In the peroral preparation, food and drink, feed, food additive or feed additive for prevention and treatment of atopic dermatitis according to the present invention, as the hydroxyproline or N-acylated derivative of hydroxyproline or a salt thereof, that cis/trans-4-hydroxy-L/D-proline, cis/trans-3-hydroxy-L/D-proline or various N-acylated derivatives thereof or salts thereof may be used either solely or as a mixture.

The amount of hydroxyproline or N-acylated derivative of hydroxyproline or a salt thereof in the peroral preparation, food and drink, feed, food additive or feed additive for prevention and treatment of atopic dermatitis according to the present invention may be increased or decreased within a broad range depending upon the aimed effect and it is, for example, 0.1 to 90% by weight, preferably 1 to 70% by weight and, particularly preferably, 5 to 50% by weight.

In addition to the above-mentioned essential component, the peroral preparation, food and drink, feed, food additive or feed additive for prevention and treatment of atopic dermatitis according to the present invention may appropriately contain additives which are suitable for each of the uses.

The peroral preparation of the present invention contains hydroxyproline or N-acylated derivative of hydroxyproline or a salt thereof and, if necessary, it may contain one or more pharmacologically acceptable carrier(s) if necessary and may contain other effective ingredients for the treatment if further necessary.

The peroral preparation of the present invention may be produced by, if necessary, mixing hydroxyproline or N-acylated derivative of hydroxyproline or a salt thereof with a carrier according to any method which has been well known in the technical field of pharmaceutical preparations.

In making the peroral preparation of the present invention into pharmaceutical preparations, it is possible to use additives such as excipient, binder, disintegrating agent, lubricant, dispersing agent, suspending agent, emulsifier, diluting agent, buffer, antioxidant and microorganism suppressor, etc.

Examples of dosage form of the peroral preparation include tablet, diluted powder, granule, emulsion, syrup and capsule, etc. The example, when the dosage form of the peroral preparation is tablet, diluted powder, granule or the like, it is possible to produce the preparation by addition of excipient such as saccharide (for example, lactose, sugar, glucose, sucrose, mannitol, sorbitol, etc.), starch (for example, that of potato, wheat, corn, etc.), inorganic substance (for example, calcium carbonate, calcium sulfate, sodium hydrogen carbonate, sodium chloride, etc.), plant powder (for example, licorice powder, gentian powder, etc.) and the like, disintegrating agent such as starch, agar, gelatin powder, crystalline cellulose, carmellose sodium, carmellose calcium, calcium carbonate, sodium hydrogen carbonate, sodium alginate, etc., lubricant such as magnesium stearate, talc, hydrogenated plant oil, Macrogol, silicone oil, etc., binder such as polyvinyl alcohol, hydroxypropyl cellulose, methyl cellulose, ethyl cellulose, carmellose, gelatin, starch paste solution, etc. , surfactant such as fatty acid ester, etc., plasticizer such as glycerol, etc. and the lile.

When the dosage form of the peroral preparation is liquid preparation such as syrup, etc. the preparation is able to be produced by adding water, saccharide such as sucrose, sorbitol, fructose, etc., glycol such as polyethylene glycol, propylene glycol, etc., oil such as sesame oil, olive oil, soybean oil, etc., antiseptic such as p-hydroxybenzoate, etc., flavor such as straw berry flavor, peppermint, etc. and the like thereto.

The concentration of hydroxyproline or N-acylated derivative of hydroxyproline or a salt thereof in the peroral preparation of the present invention may be appropriately selected depending upon the type of the peroral preparation, the effect expected by administration of the peroral preparation, etc. and, usually, it is 0.1 to 90% by weight, preferably 1 to 70% by weight or, particularly preferably, 5 to 50% by weight as hydroxyproline or N-acylated derivative of hydroxyproline or a salt thereof.

Dose of the peroral preparation of the present invention varies depending upon dosage form, age and body weight of a person to be administered, etc. and, usually, hydroxyproline or N-acylated derivative of hydroxyproline or a salt thereof is administered in an effective amount of 100 to 10,000 mg, preferably 100 to 2,000 mg or, particularly preferably, 200 to 1,000 mg per day for an adult once daily or by dividing into several times a day.

The peroral preparation of the present invention may be used together with other anti-itching agent such as steroidal preparation, etc.

With regard to the steroidal preparation, glucocorticosteroidal preparation such as prednisolone, cortisol, dexamethasone, betamethasone, etc. and salt steroidal preparation such as fludrocortisone, aldosterone, etc. and the like are used and glucocorticosteroidal preparation is preferably used.

"Prevention of atopic dermatitis" in the present invention means that, when a preventive agent for atopic dermatitis of the present invention is administered or ingested on a daily basis, such effects that (a) onset of atopic dermatitis is completely prevented, (b) rate of onset thereof is reduced or (c) symptom upon onset thereof is suppressed are achieved.

The food additive of the present invention is able to be prepared by the same method as in the peroral preparation mentioned above. Usually, the food additive is mixed or dissolved with other food additive if necessary and is processed/manufactured into a form such as powder, granule, pellet, tablet and various liquid preparations.

With regard to the food and drink of the present invention, one in which N-acylated derivative of hydroxyproline or a salt thereof is added to food and drink may be exemplified.

The food and drink of the present invention may be processed/produced according to a common method for the production of food and drink except that hydroxyproline or N-acylated derivative of hydroxyproline or a salt thereof is added to food and drink.

The food and drink of the present invention may also be produced by means of a granulating method such as fluidized bed granulation, stirring granulation, extrusion granulation, tumbling granulation, air current granulation, compression molding granulation, disintegration granulation, spray granulation, jet granulation, etc., a coating method such as pan coating, fluidized bed coating, dry coating, etc., a swelling method such as puff dry, excessive steam method, foam mat method, microwave heating method, etc., an extrusion method using extrusion granulator, extruder, etc. and the like.

The food and drink of the present invention may be in any of forms of juice, refreshing drink, tea, lactic acid bacteria beverage, milk product such as fermented milk, ice cream, butter, cheese, yogurt, processed milk, skim milk, etc., meat product such as ham, sausage, hamburger, etc., fish meat paste product such as *kamaboko* (boiled fish paste), *chikuwa* (Japanese fish sausage), *Satsuma-age* (deep-fried fish ball containing vegetable bits), etc., egg product such as *dashi-maki* (Japanese rolled omelet), steamed egg custard, etc., confectionery such as cookie, jelly, chewing gum, candy, snack, etc., bread, noodle, pickles, smoked product, dried product, *tsukudani* (fish boiled down with soy), salted product, soup, seasoning, etc.

The food and drink of the present invention may also be in a form such as powdery food, sheet food, bottled food, canned food, retort food, capsule food, tablet food, fluid food, drink food, etc.

The food and drink of the present invention may also be used as health food, functional food or the like for prevention or treatment of atopic dermatitis.

The food additive which is commonly used for food and drink such as sweetener, coloring agent, preservative, thickener/stabilizer, antioxidant, color coupler, bleaching agent, antifungal agent, gum base, bitter agent, enzyme, brightener, acidifying agent, seasoning, emulsifier, enriching agent, agent for manufacture, flavor ingredient, spice and the like extract may be added to the food and drink or the food additive of the present invention.

The amount of hydroxyproline or N-acylated derivative of hydroxyproline or a salt thereof or the food additive of the present invention to be added to the food and drink of the present invention is appropriately selected depending upon type of food and drink, effect expected by ingestion of the food and drink, etc. and, as hydroxyproline or N-acylated derivative of hydroxyproline or a salt thereof, it is usually added so as to make its content 0.1 to 90% by weight, preferably 1 to 70% by weight or, more preferably, 5 to 50% by weight.

The amount of the food and drink of the present invention to be ingested varies depending upon ingesting form, age, body weight, etc. of the person who ingests it, and it is usually 100 to 10,000 mg, preferably 100 to 2,000 mg or, particularly preferably, 200 to 1,000 mg as hydroxyproline or N-acylated derivative of hydroxyproline or a salt thereof per day for an adult as an effective amount and it is ingested once daily or by dividing into several times a day. Although there is no particular limitation for the ingesting period, it is usually from one day to one year or, preferably, from one week to three months.

The feed additive of the present invention may be prepared by the same method as the peroral preparation of the present invention. Usually, the feed additive is mixed or dissolved with other feed additive if necessary and is processed/produced into a form of, for example, powder, granule, pellet, tablet and various liquid preparations.

The feed of the present invention may be any feed such as feed for pets, feed for livestock, feed for domestic fowls, etc. so far as it is a feed for prevention or treatment of atopic dermatitis of animals such as mammals, birds, etc and it is preferably used as a feed for prevention or treatment of atopic dermatitis of pets such as monkey, dog, cat, rat, mouse, etc.

The feed of the present invention may be processed/produced by a common method for the production of feed except that hydroxyproline or N-acylated derivative of hydroxyproline or a salt thereof or the feed additive of the present invention is added to the feed.

Examples of the feed are cereal, chaff and bran, plant oil cake, feed derived from animals and other feed, purified product, a mixture thereof or the like.

Examples of cereal include milo, wheat, barley, oat, rye, unpolished rice, buckwheat, foxtail millet, broomcorn millet,
Japanese millet, corn, soybean, etc.

Examples of chaff and bran include rice bran, defatted rice bran, wheat bran, rice flour, wheat, embryo, barley bran, pellet, corn bran, corn embryo, etc.

Examples of plant oil cake include soybean oil cake, soybean flour, linseed oil cake, cottonseed oil cake, peanut oil cake, safflower oil cake, coconut oil cake, palm oil cake, sesame oil cake, sunflower oil cake, rapeseed oil cake, kapok oil cake, mustard oil cake, etc.

Examples of feed derived from animals include fish powder such as Northern ocean meal, imported meal, whole meal, coast meal, etc. , fish soluble, meat powder, meat bone powder, blood powder, degraded hair, bone powder, side product upon treatment of animals, feather meal, chrysalis of silk worm, skim milk, casein, dry whey and the like.

Examples of other feed include plant stems/leaves such as alfalfa, hay cube, alfalfa leaf meal, black locust powder, etc., side products in corn process industry such as corn gluten, meal, corn gluten feed, corn steep liquor, etc., processed starch such as starch, etc., fermentation industry product such as yeast, beer cake, malt root, alcohol cake, soy sauce cake, etc., side product in agricultural manufacture such as cake after processing of citrus fruits, soybean curd cake, coffee grounds, cocoa grounds, etc., cassava, broad bean, guarmeal, sea algae, krill, spirulina, chlorella, minerals and the like.

Examples of pure product include protein such as casein, albumin, etc., amino acids, starch, cellulose, saccharide such as sucrose, glucose, etc., minerals, vitamins and the like.

The feed of the present invention is also able to be produced by a granulation method such as fluidized bed granulation, stirring granulation, extrusion granulation, tumbling granulation, air current granulation, compression molding granulation, disintegrating granulation, spray granulation, jet granulation, etc. , coating method such as pan coating, fluidized bed coating, dry coating, etc., swelling method such as puff dry, excessive steam method, foam mat method, microwave heating method, etc., extrusion method using extrusion granulator, extruder, etc. and the like.

The amount of hydroxyproline or N-acylated derivative of hydroxyproline or a salt thereof or a feed additive to be added to the feed of the present invention is appropriately selected depending upon type of feed, effect expected by ingestion of the feed, etc. and, as hydroxyproline or N-acylated derivative of hydroxyproline or a salt thereof, it is usually added so as to make its content 0.1 to 90% by weight, preferably 1 to 70% by weight or, more preferably, 5 to 50% by weight.

The amount of the feed of the present invention to be ingested varies depending upon ingesting form, type of the animal which ingests it, age and body weight of the animal which ingests it, etc. and it is usually 100 to 10,000 mg, preferably 100 to 2,000 mg or, particularly preferably, 200 to 1,000 mg as hydroxyproline or N-acylated derivative of hydroxyproline or a salt thereof per day for the animal as an effective amount and it is ingested once daily or by dividing into several times a day. Although there is no particular limitation for the ingesting period, it is usually from one day to one year or, preferably, from one week to three months.

As hereunder, test examples where preventive or therapeutic effect for atopic dermatitis by hydroxyproline or N-acylated derivative of hydroxyproline are shown.

Test Example 1: Evaluation of suppression of auricular edema using model mice where atopic dermatitis appeared

For the test, NC/Nga mice (five weeks age; purchased from Charles River) were used. The breeding conditions were that the mice were bred under room temperature of $22 \pm 2°C$ and humidity of $35 \pm 15\%$ and that feed and water were freely taken by them.

Since nine days before application of hapten, administration of the test feed as shown in Table 1 was started. The test feed was prepared by well mixing of powdery feed CE-2 (manufactured by Nippon Claire) with 0.25% by weight of N-acetyl-trans-4-hydroxy-L-proline or 0.50% by weight of trans-4-hydroxy-L-proline. The feed containing N-acetyl-trans-4-hydroxy-L-proline was named a feed 1 while the feed containing trans-4-hydroxy-L-proline was named a feed 2 and, as a control feed, a powdery feed CE-2 was used.

Table 1

| Component | Feed 1 (% by weight) | Feed 2 (% by weight) |
|---|---|---|
| Powdery Feed CE-2 | 99.75 | 99.5 |
| N-acetyl-trans-4-hydroxy-L-proline | 0.25 | 0 |
| trans-4-hydroxy-L-proline | 0 | 0.5 |

With regard to the hapten, a product prepared by dissolving 1.5% by weight of dinitrochlorobenzene (DNCB) in a solution comprising 20% by volume of olive oil and 80% by volume of acetone was used.

Three mice were used for each of the test groups, and 20 μl of 1.5% DNCB was respectively applied onto both side of the right auricle of each of the mice after nine days, ten days, thirteen days and fourteen days from initiation of administration of the test feed.

Starting from the day when application of the hapten started, thickness of the right auricle was measured using Pescock Dial Thickness Gauge (manufactured by Ozaki Seisakusho) one daily.

An increasing amount of auricular edema was calculated according to the following formula 1 as a relative value to an increasing amount of auricular edema of a mouse which was not treated with DNCB and the result is shown in Table 2.

(Formula 1)

Relative increasing amount of auricular edema (%) = [(A1

$\times$ B2)/(A2 $\times$ B1)] $\times$ 100

A1: Thickness of auricle of the testing mouse after predetermined days
A2: Thickness of auricle of the testing mouse when the test was started
B1: Thickness of auricle of the untreated mouse after predetermined days
B2: Thickness of auricle of the untreated mouse when the test was started

Table 2

| Treatment with DNCB | Feed | Increased Amount (%) Relative Auricular Edema | |
|---|---|---|---|
| | | On the 23rd Day | On the 27th Day |
| - | CE-2 | $100.0 \pm 2.7$ | $100.0 \pm 2.7$ |
| + | CE-2 | $642.0 \pm 23.7$ | $478.9 \pm 38.0$ |
| + | Feed 1 | $565.7 \pm 40.5$ | $390.2 \pm 37.0$ |
| + | Feed 2 | $510.7 \pm 78.2$ | $369.8 \pm 62.6$ |

In the control feed group, auricular edema was induced by DNCB, while in the group of a feed containing 0.25% of N-acetyl-trans-4-hydroxy-L-proline and the group of a feed containing 0.5% of trans-4-hydroxy-L-proline, auricular edema was apparently suppressed.

Test Example 2: Evaluation of IgE concentration using atopic dermatitis model mice

With regard to the model mice where atopic dermatitis appeared used in Test Example 1, blood was collected from right thigh on the 27th day from initiation of administration of the test feed. The collected blood sample was centrifuged, serum was recovered and the result of IgE concentration in blood by means of an ELISA is shown in Table 3.

Table 3

| Treatment with DNCB | Feed | IgE Concentration in Serum (ng/ml) |
|---|---|---|
| - | CE-2 | 62.5 ± 20.8 |
| + | CE-2 | 833.3 ± 128.1 |
| + | Feed 1 | 365.7 ± 103.4 |
| + | Feed 2 | 441.0 ± 274.3 |

In the control feed groups, IgE concentration in serum increased by application of DNCB, while in the group of a feed containing 0.25% of N-acetyl-trans-4-hydroxy-L-proline and the group of a feed containing 0.5% of trans-4-hydroxy-L-proline, increase in IgE concentration in serum by application of DNCB was significantly suppressed.

Test Example 3: Evaluation of mast cells using model mice where atopic dermatitis appeared

With regard to the model mice where atopic dermatitis appeared used in Test Example 1, tissues at auricle were frozen and embedded in Tissue-Tek O. C. T. Compound (manufactured by Sakura Seiki K. K.) on the 27th day from initiation of administration of the test feed. Thin slices of 6 μm were prepared using a cryostat and stained with Toluidine Blue and the result of counting the mast is shown in Table 4.

Table 4

| Treatment with DNCB | Feed | Mast Cell Density (cells/mm$^2$) |
|---|---|---|
| - | CE-2 | 9.8±5.8 |
| + | CE-2 | 33.2±10.8 |
| + | Feed 1 | 17.1±2.1 |
| + | Feed 2 | 17.4±2.3 |

In the control feed groups, mast cell density at inflammation site increased by application of DNCB, while in the group of a feed containing 0.25% of N-acetyl-trans-4-hydroxy-L-proline and the group of a feed containing 0.5% of trans-4-hydroxy-L-proline, increase in mast cell density by application of DNCB was apparently suppressed.
From the above, it is shown that oral ingestion of hydroxyproline or N-acylated derivative of hydroxyproline is effective for prevention or treatment of atopic dermatitis.
Examples of the present invention are shown as follows.

BEST MODE FOR CARRYING OUT THE INVENTION

Example 1: Preparation of tablets containing N-acylated derivative of hydroxyproline

Tablets containing N-acetyl-trans-4-hydroxy-L-proline were prepared according to a conventional method. Thus, the components mentioned in Table 5 were uniformly mixed and the mixture was tabletted using a single-shot tabletting machine to prepare tablets each having 5 mm diameter and 15 mg weight.

Table 5

| Components | Amounts (g) |
|---|---|
| N-acetyl-trans-4-hydroxy-L-proline | 10.0 |
| Lactose | 90.0 |
| Dry corn starch | 2.0 |
| Talc | 1.8 |
| Magnesium stearate | 0.2 |

Example 2: Preparation of granules containing N-acylated derivative of hydroxyproline

Tablets prepared in Example 1 were disintegrated, granulated and sieved to give granular preparation of 20 to 50 meshes.

Example 3: Preparation of beverage containing N-acylated derivative of hydroxyproline

Beverage containing N-acetyl-trans-4-hydroxy-L-proline was prepared by uniformly stirring and dissolving the components mentioned in Table 6 and by adding purified water thereto so as to make the total amount 1,000 ml. The term reading "q. s." in Table 6 means an amount used for common beverage in the case of flavoring ingredient and pigment and, in the case of purified water, it means an amount necessary for making the total amount 1,000 ml together with other components.

Table 6

| Components | Amounts (g) |
|---|---|
| N-acetyl-trans-4-hydroxy-L-proline | 5.0 |
| Sodium Benzoate | 1.0 |
| Fructose | 10.0 |
| Flavoring ingredient | q. s. |
| Pigment | q. s. |
| Purified Water | q. s. |

Example 4: Preparation of candy containing N-acylated derivative of hydroxyproline

Candy comprising the components mentioned in Table 7 including N-acetyl-trans-4-hydroxy-L-proline was prepared by a conventional method.

Table 7

| Components | Amounts (g) |
|---|---|
| N-acetyl-trans-4-hydroxy-L-proline | 1.0 |
| Powdery sorbitol | 98.75 |
| Flavoring ingredient | 0.2 |
| Sorbitol seed | 0.05 |

INDUSTRIAL APPLICABILITY

In accordance with the present invention, there is provided a safe peroral preparation, food and drink, feed, food additive or feed additive for prevention or treatment of atopic dermatitis.

**Claims**

1. A peroral preparation for prevention or treatment of atopic dermatitis comprising hydroxyproline or N-acylated derivative of hydroxyproline or a salt thereof.

2. The peroral preparation according to claim 1, wherein it comprises 0.1 to 90% by weight of hydroxyproline or N-acylated derivative of hydroxyproline or a salt thereof.

3. The peroral preparation according to claim 1 or 2, wherein the acyl group of N-acylated derivative of hydroxyproline is an acyl group having 1 to 24 carbon atoms.

4. The peroral preparation according to any one of claims 1 to 3, wherein the N-acylated derivative of hydroxyproline is N-acetylated derivative, N-propionylated derivative, N-butyrylated derivative or isobutyrylated derivative.

5. Food and drink or feed for prevention or treatment of atopic dermatitis comprising hydroxyproline or N-acylated derivative of hydroxyproline or a salt thereof.

6. The food and drink or feed according to claim 5, wherein it comprises 0.1 to 90% by weight of hydroxyproline or N-acylated derivative of hydroxyproline or a salt thereof.

7. The food and drink or feed according to claim 5 or 6, wherein the acyl group of N-acylated derivative of hydroxyproline is an acyl group having 1 to 24 carbon atoms.

8. The food and drink or feed according to any one of claims 5 to 7, wherein the N-acylated derivative of hydroxyproline is N-acetylated derivative, N-propionylated derivative, N-butyrylated derivative or isobutyrylated derivative.

9. Food additive or feed additive for prevention or treatment of atopic dermatitis comprising hydroxyproline or N-acylated derivative of hydroxyproline or a salt thereof.

10. The food additive or feed additive according to claim 9, wherein it comprises 0.1 to 90% by weight of hydroxyproline or N-acylated derivative of hydroxyproline or a salt thereof.

11. The food additive or feed additive according to the claim 9 or 10, wherein the acyl group of N-acylated derivative of hydroxyproline is an acyl group having 1 to 24 carbon atoms.

12. The food additive or feed additive according to any one of claims 9 to 11, wherein the N-acylated derivative of hydroxyproline is N-acetylated derivative, N-propionylated derivative, N-butyrylated derivative or isobutyrylated derivative.

13. A method of preventing or treating atopic dermatitis, which comprises orally administering or ingesting hydroxyproline or N-acylated derivative of hydroxyproline or a salt thereof in an effective amount.

14. Use of hydroxyproline or N-acylated derivative of hydroxyproline or a salt thereof for the manufacture of peroral preparation, food, feed, food additive or feed additive for prevention or treatment of atopic dermatitis.

<div align="center">

**INTERNATIONAL SEARCH REPORT**

</div>

| | International application No. |
|---|---|
| | PCT/JP03/14022 |

| A. CLASSIFICATION OF SUBJECT MATTER |
|---|
| Int.Cl$^7$ A61K31/401, A61P17/00, 37/08 |

According to International Patent Classification (IPC) or to both national classification and IPC

| B. FIELDS SEARCHED |
|---|
| Minimum documentation searched (classification system followed by classification symbols) |
| Int.Cl$^7$ A61K31/401 |

Documentation searched other than minimum documentation to the extent that such documents are included in the fields searched

Electronic data base consulted during the international search (name of data base and, where practicable, search terms used)
CA(STN), REGISTRY(STN), WPIDS(STN)

**C. DOCUMENTS CONSIDERED TO BE RELEVANT**

| Category* | Citation of document, with indication, where appropriate, of the relevant passages | Relevant to claim No. |
|---|---|---|
| A | WO 02/06225 A (Kyowa Hakko Kogyo Co., Ltd.), 24 January, 2002 (24.01.02), Full text & EP 1304323 A | 1-12,14 |

☐ Further documents are listed in the continuation of Box C. ☐ See patent family annex.

| | |
|---|---|
| * Special categories of cited documents: <br> "A" document defining the general state of the art which is not considered to be of particular relevance <br> "E" earlier document but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| Date of the actual completion of the international search <br> 02 December, 2003 (02.12.03) | Date of mailing of the international search report <br> 16 December, 2003 (16.12.03) |
| Name and mailing address of the ISA/ <br> Japanese Patent Office | Authorized officer |
| Facsimile No. | Telephone No. |

Form PCT/ISA/210 (second sheet) (July 1998)

<table>
<tr><td>**INTERNATIONAL SEARCH REPORT**</td><td>International application No.<br>PCT/JP03/14022</td></tr>
</table>

**Box I     Observations where certain claims were found unsearchable (Continuation of item 2 of first sheet)**

This international search report has not been established in respect of certain claims under Article 17(2)(a) for the following reasons:

1. ☒ Claims Nos.: 13

   because they relate to subject matter not required to be searched by this Authority, namely:
   The invention as set forth in claim 13 pertains to method for treatment of the human body by therapy.

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II     Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐     The additional search fees were accompanied by the applicant's protest.

☐     No protest accompanied the payment of additional search fees.

Form PCT/ISA/210 (continuation of first sheet (1)) (July 1998)